# EUROPÄISCHE PATENTSCHRIFT

(11) **EP 0 951 879 B1**
(45) Veröffentlichungstag und Bekanntmachung des Hinweises auf die Patenterteilung: **07.05.2008**
(21) Anmeldenummer: 99106986.5
(22) Anmeldetag: 09.04.1999
(51) Int. Cl.: A61F 2/44

(54) **Vorrichtung zur Verbindung von Wirbeln der Wirbelsäule**
Device for connecting vertebrae of the spinal column
Dispositif pour fusionner des vertèbres de la colonne vertébrale

(30) Priorität: 23.04.1998 DE 19818143
(43) Veröffentlichungstag der Anmeldung: 27.10.1999
(73) Patentinhaber: MBA INCORPORADO S.A., 33204 Gijon Asturias (ES)
(72) Erfinder: Weiland, Peter, 66620 Nonnweiler-Braunshausen (DE); Wilke, Hans-Joachim, Priv.-Doz. Dr., 89160 Dornstadt (DE)
(74) Vertreter: Bernhardt, Winfrid

(56) Entgegenhaltungen:
- EP-A- 0 697 200
- WO-A-97/15246
- WO-A-98/02117
- US-A- 5 683 394

## Beschreibung

Die Erfindung betrifft eine Vorrichtung zur Verbindung von Wirbeln der Wirbelsäule über ein zwischen den Wirbeln anzuordnendes Implantat, das wenigstens einen Hohlraum und Öffnungen zur Durchsetzung des Implantats mit zur Verbindung der Wirbel gebildetem Knochengewebe aufweist, wobei das Implantat einen hohlen, im wesentlichen rotationssymmetrischen Verbindungsteil für die Anlage gegen freigelegtes Knochenweichgewebe mit einer sich senkrecht zur Wirbelsäulenlängsachse erstreckenden Symmetrieachse aufweist, von dem sich diametral Abstandhalterflügel für die Anlage gegen Knochenhartgewebe erstrecken.

Bei der Verbindung von Wirbeln, z.B. nach einer Bandscheibenresektion, stabilisiert ein solches Implantat die Wirbelsäule, solange sich noch nicht genügend Knochenverbindungsgewebe gebildet hat.

Es sind in ihren Umrissen zylindrische Implantate mit einem Außengewinde bekannt, die in eine vorbereitete Gewindebohrung eingedreht werden, welche Gewindeabschnitte in den im Abstand einander gegenüberliegenden, zu verbindenden Wirbeln aufweist. Beim Einbringen der Gewindeabschnitte wird die Knochenhartgewebeschicht so weit abgetragen, dass die zylindrischen Implantate über einen Teil ihres Umfangs in Kontakt mit dem darunterliegenden Knochenweichgewebe kommen. Allein das Knochenweichgewebe ist in der Lage, Knochenverbindungsgewebe zu bilden, das durch Öffnungen in der Zylinderwand des Implantats eindringen und das Implantat durchsetzen kann.

Es sind ferner in ihren Umrissen quaderförmige Implantate bekannt, bei deren Implantation in den gegenüberliegenden Wirbelstirnseiten entsprechende Einfräsungen vorzunehmen sind, um zu ermöglichen, dass durch freigelegtes Knochenweichgewebe Verbindungsgewebe gebildet werden und das Implantat durchsetzen kann.

Ein Implantat der eingangs genannten Art geht aus der US 5 683 394 und der WO 98/02117 hervor. Dieses Implantat weist auf seinen dem Knochenweichgewebe zugewandten Seiten des Verbindungsteils jeweils eine sich in ihrer Längsausdehnung parallel zur Rotationssymmetrieachse erstreckende Öffnung auf. Die Öffnungen sind durch eine durchgehende Ausnehmung gebildet. Durch ein solches Implantat kann die Wirbelsäule vertikal belastet werden, wobei die gegen Knochenhartgewebe anliegenden Abstandhalterflügel dafür sorgen, dass die Wirbel ihre erforderliche Position innerhalb der Wirbelsäule beibehalten. Bei Implantaten ohne solche Abstandhalterflügel kann es leicht vorkommen, dass das Implantat in das Knochenweichgewebe hineingedrückt wird und sich der Abstand zwischen den Wirbeln unerwünscht verringert.

Durch die vorliegende Erfindung wird eine neue Vorrichtung zur Verbindung von Wirbeln der Wirbelsäule der obengenannten Art geschaffen, welche gegenüber herkömmlichen solchen Vorrichtungen die Wirbelsäule während des Wachstums des Knochenverbindungsgewebes besser stabilisiert und eine exaktere Lagefixierung der Wirbel innerhalb der Wirbelsäule ermöglicht.

Die diese Aufgabe lösende Vorrichtung nach der Erfindung ist dadurch gekennzeichnet, dass sich der Hohlraum in die Abstandshalterflügel hinein erstreckt und die Vorrichtung auf ihren dem Knochengewebe zugewandten Seiten jeweils mehrere, sich in ihrer Längsausdehnung senkrecht zu der Symmetrieachse erstreckende Öffnungsschlitze aufweist, die sich über den Verbindungsteil und die Abstandhalterflügel erstrecken.

Vorteilhaft tritt durch diese Öffnungsschlitze sich bildendes Knochenverbindungsgewebe in den Hohlkörper ein und hintergreift unter Arretierung des Implantats Wandabschnitte des Hohlkörpers.

In einer bevorzugten Ausführungsform der Erfindung ist der Verbindungsteil als Zylinder oder Konusabschnitt ausgebildet. In einem solchen Fall läßt sich die zur Freilegung des Knochenweichgewebes erforderliche Knochenbearbeitung mit einem Bohr- oder Fräswerkzeug durchführen, wobei die Maße der gebildeten Ausbohrungen oder Ausfräsungen im wesentlichen durch die Maße des Werkzeugs bestimmt sind. Zur Knochenbearbeitung braucht das Werkzeug lediglich um einen erforderlichen Betrag vorgeschoben zu werden, und es ist zur Bearbeitung darüber hinaus keinerlei Werkzeugbewegung erforderlich.

Während der Verbindungsteil in Kontakt mit Weichknochengewebe in eine Ausbohrung oder Ausfräsung hineinsteht, liegen die sich zu beiden Seiten des Verbindungsteils erstreckenden Abstandhalterflügel jeweils gegen Knochenhartgewebe des oberen und unteren Wirbels an, welches an die jeweiligen Ausbohrungen oder Ausfräsungen angrenzt.

In einer weiteren Ausführungsform der Erfindung ist vorgesehen, dass sich der Verbindungsteil und die Abstandhalterflügel entsprechend geneigt zueinander angeordneten Wirbeln verjüngen. Diese Ausführungsform ist zur Verbindung solcher Wirbel vorgesehen, welche gebogenen Abschnitten der Wirbelsäule angehören.

In einer weiteren bevorzugten Ausführungsform der Erfindung weist der Hohlkörper auf einer Stirnseite eine insbesondere mit Durchbrüchen versehene Stirnwand auf und ist auf der anderen Stirnseite offen. Ein solcher Hohlkörper läßt sich mit der geschlossenen Stirnwandseite nach vom in den Zwischenraum zwischen den zu verbindenden Wirbeln einführen, wobei über die offene andere Stirnseite z.B. Halterungswerkzeuge in den Implantathohlkörper einführbar sind. Die Stirnwand dient auch zur Fixierung von Füllmaterial, das zur Förderung der Durchbauung mit Knochengewebe in den Hohlkörper einfüllbar ist.

In weiterer Ausgestaltung der Erfindung ist eine Arretiereinrichtung zur Festlegung des Implantats quer zur Wirbelsäulenlängsrichtung vorgesehen, die dafür sorgt, dass das Implantat nicht aus dem Zwischenraum zwischen den zu verbindenden Wirbeln austreten kann. Eine solche Arretierungseinrichtung kann einen in das Implantat einführbaren Einsatz mit einer Verhakungseinrichtung, die insbesondere in das Knochenweichgewebe eingreift, umfassen.

In einer bevorzugten Ausführungsform weist die Verhakungseinrichtung Hakenteile auf, die an einem in der Art eines Zylinderschlosses in dem Implantat drehbaren Einsatzkörper angebracht sind, wobei die Hakenteile durch die Öffnungsschlitze hindurch in das Knochenweichgewebe eindrehbar sind. Ein solcher Einsatz mit Hakenteilen lässt sich in das Implantat einschieben, wobei sich die Hakenteile innerhalb der Abstandhalterflügel bewegen. Durch die sich bis auf die Abstandhalterflügel erstreckenden Öffnungsschlitze ist gewährleistet, daß die Hakenteile bei Drehung des Einsatzkörpers durch die Öffnungsschlitze hindurch in das Knochenweichgewebe hinein vortreten und so für eine Verhakung des Implantats sorgen können.

Der Einsatz kann in dem Implantat zwischen der genannten Stirnwand und einer an der anderen Stirnseite anbringbaren Sperre festlegbar sein, wobei als Sperre vorzugsweise eine in dem Implantat versenkbare, kopflose Schraube verwendet wird.

Vorteilhaft kann der Einsatz in dem Implantat mit einem vorgegebenen Spiel festgelegt sein, das so gering ist, daß es die genaue Positionierung der Wirbel nicht beeinträchtigt, die geringfügigen Bewegungen des mit dem Knochenweichgewebe verhakten Arretierungseinsatzes aber das Wachstum von Knochenverbindungsgewebe anregen.

Die Erfindung soll nun anhand von Ausführungsbeispielen und der beiliegenden, sich auf diese Ausführungsbeispiele beziehenden Zeichnungen näher erläutert und beschrieben werden. Es zeigen:
- Fig. 1: ein Implantat gemäß der Erfindung in einer perspektivischen Darstellung,
- Fig. 2: das Implantat von Fig. 1 in einer Seitenansicht,
- Fig. 3: das Implantat von Fig. 1 in einer Schnittdarstellung gemäß Schnittlinie A-A von Fig. 2,
- Fig. 4: das Implantat von Fig. 1 in einer Schnittdarstellung gemäß Schnittlinie B-B von Fig. 2,
- Fig. 5: das Implantat von Fig. 1 in einer Schnittdarstellung gemäß Schnittlinie C-C von Fig. 2 in einer Anordnung zwischen zwei Wirbeln,
- Fig. 6: das Implantat von Fig. 1 in der Ansicht von Fig. 2 in einer Anordnung zwischen zwei, durch eine Halterungseinrichtung positionierten Wirbeln,
- Fig. 7: ein weiteres Ausführungsbeispiel für ein erfindungsgemäßes Implantat mit einem Arretierungseinsatz, und
- Fig. 8: den in dem Implantat von Fig. 7 verwendeten Arretierungseinsatz in einer perspektivischen Darstellung.

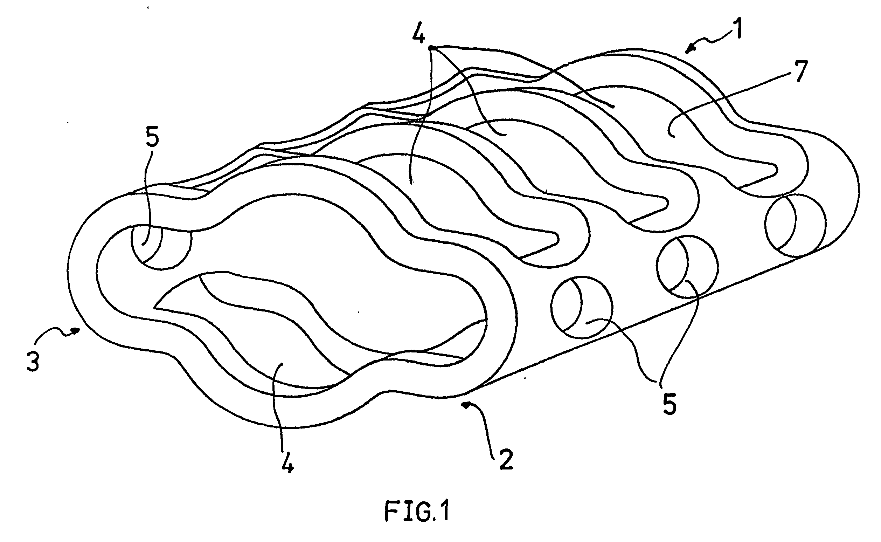

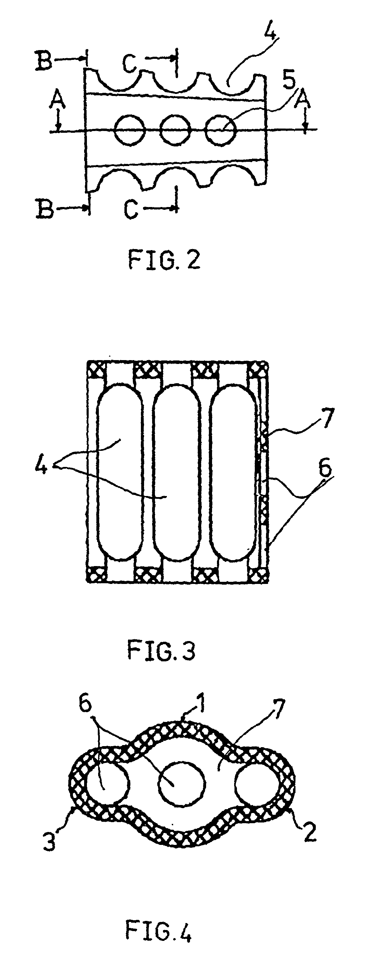

Ein in den Fig. 1 bis 6 gezeigter Implantathohlkörper weist einen in seinen Umrissen etwa zylindrisch geformten Verbindungsteil 1 auf, von dem diametral Abstandhalterflügel 2 und 3 vorstehen.

Die Längswände des Implantathohlkörpers sind in der Art eines Käfigs durchbrochen, wobei insbesondere einander gegenüberliegende, sich quer zur Längsachse des Verbindungsteils 1 erstreckende Öffnungsschlitze 4 vorgesehen sind.

In Fig. 1 sind die Schlitze 4 auf der Unterseite des Hohlkörpers der besseren Übersichtlichkeit halber nur zum Teil eingezeichnet.

Wie den Figuren zu entnehmen ist, erstrecken sich die Schlitze 4 nicht nur in der Wand des Verbindungskörpers 1 sondern auch der Abstandhalterflügel 2 und 3.

An den Flügelenden der Abstandhalterflügel 2 und 3 sind jeweils Durchbrüche 5 vorgesehen.

Der Implantathohlkörper ist auf einer Stirnseite offen. Auf der der offenen Stirnseite entgegengesetzten Stirnseite ist eine Durchbrüche 6 aufweisende Stirnwand 7 gebildet.

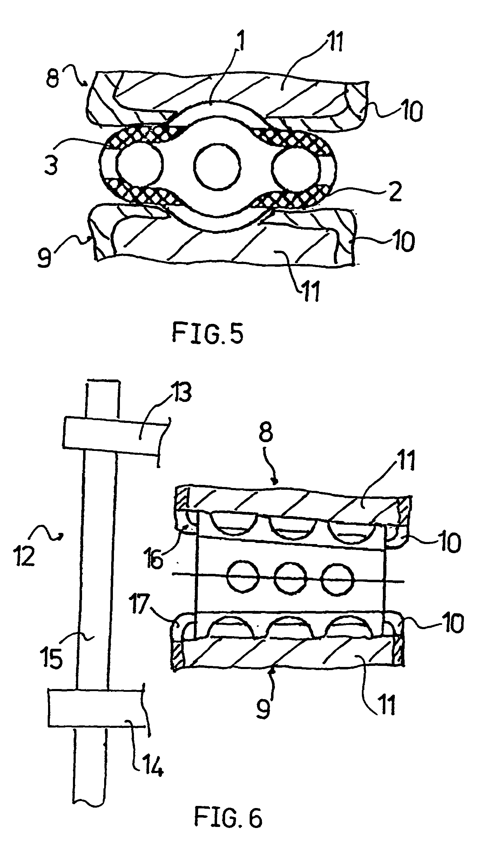

Es wird nun insbesondere auf die Fig. 5 und 6 Bezug genommen, wo mit 8 und 9 Abschnitte miteinander zu verbindender Wirbel bezeichnet sind. Die Wirbel weisen eine äußere Knochenhartgewebeschicht 10 und eine darunterliegendes Knochenweichgewebe 11 auf.

Zum Einsetzen des Implantats zwischen den Wirbeln 8 und 9 werden die Wirbel über eine bei 12 schematisch angedeutete Halterung im Abstand und zueinander geneigt positioniert, wobei die Positionierung der vorgesehenen Stellung der Wirbel in einem gekrümmten Abschnitt der Wirbelsäule entspricht. Die Halterung 12 weist über nicht näher beschriebene Schraubverbindungen an den Wirbeln 8 und 9 anbringbare Halterungsteile 13 und 14 auf, die an einem Träger 15 über geeignete Befestigungseinrichtungen angebracht werden können.

In der so fixierten Stellung werden die Wirbel 8 und 9 mit Hilfe eines leicht konischen Fräsers bearbeitet, welcher zwischen den Wirbeln in Richtung seiner Drehachse vorgeschoben wird, wobei entsprechend den Maßen des Fräsers in den Wirbeln 8 und 9 einander gegenüber liegende Ausnehmungen 16 und 17 gebildet werden, in denen das Knochenweichgewebe 11 freigelegt ist.

Nach Bildung dieser Ausnehmungen 16 und 17 kann das Implantat in Richtung der Längsachse des Verbindungsteils 1 in den Zwischenraum zwischen den geneigt angeordneten Wirbeln 8 und 9 eingeschoben werden, wobei, wie aus den Fig. 5 und 6 hervorgeht, der Verbindungsteil 1 mit dem größten Teil seines freiliegenden Umfangs mit dem Knochenweichgewebe 11 in Kontakt kommt. Die Abstandhalterflügel 2 und 3 liegen gegen Knochenhartgewebe 10 an, welches im Unterschied zum Knochenweichgewebe 11 weniger nachgiebig ist. Damit kann durch das beschriebene Implantat der erforderliche Abstand zwischen den Wirbeln 8 und 9 auch bei verhältnismäßig starker vertikaler Belastung der Wirbelsäule gewährleistet werden.

In dem gezeigten Ausführungsbeispiel variiert der Durchmesser des konischen Verbindungsteils 1 etwa zwischen 8,5 und 7,5 mm. Die den Abstand zwischen den Wirbeln 8 und 9 bestimmende Dicke der Abstandhalterflügel 2 und 3 liegt in dem gezeigten Ausführungsbeispiel etwa zwischen 5 und 4 mm. Ein Implantat mit solchen Abmessungen kann zur Verbindung von Wirbeln im Halsbereich verwendet werden.

Nach der Implantation bildet das Knochenweichgewebe 11 weiteres Knochenverbindungsgewebe, das durch die Schlitze 4 von beiden Seiten in den Implantathohlkörper eindringt und den Implantathohlkörper durchsetzt, wobei es in der Regel zu einer vollständigen Durchsetzung des Implantathohlkörpers unter Überbrückung des Zwischenraums zwischen den Wirbeln 8 und 9 mit Knochengewebe kommt. Das Knochenverbindungsgewebe kann sich im gesamten Hohlraum des Implantats ausbreiten, wobei es auch die Durchbrüche 5 und 6 durchsetzen kann.

Eine Verbindung zwischen den Wirbeln 8 und 9 kann ohne Brückenbildung aber auch allein schon dadurch hergestellt sein, daß das in den Implantathohlkörper eindringende Knochengewebe die Wände des Implantathohlkörpers hintergreift.

Solange das die Wirbel verbindende Knochengewebe noch nicht gebildet ist, sorgt der Implantathohlkörper für eine Stabilisierung der Wirbelsäule, wobei insbesondere vertikale Kräfte durch das Implantat abgetragen werden.

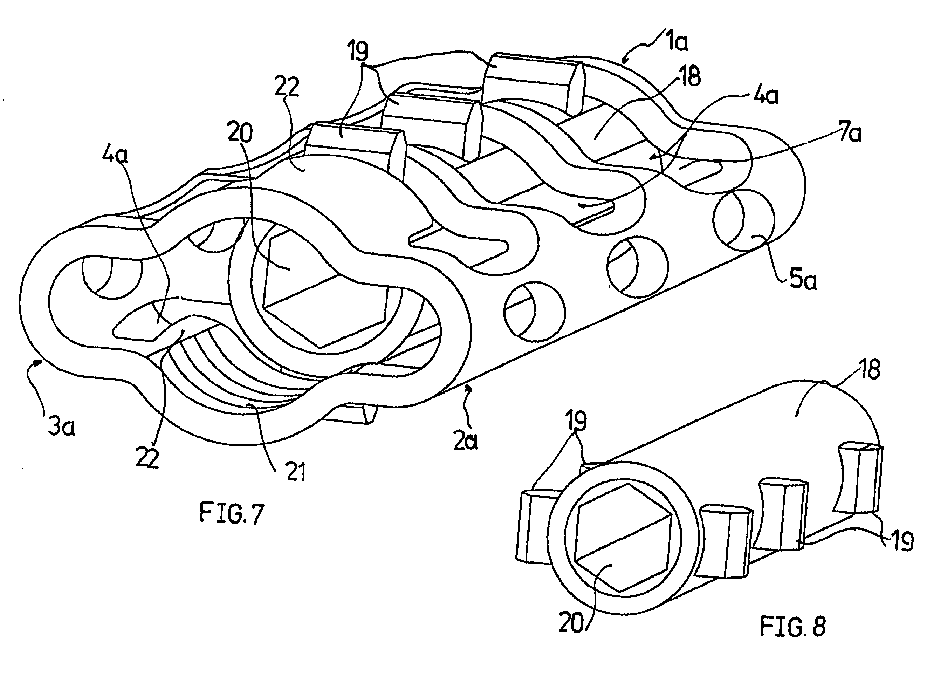

Es wird nun auf Fig. 7 und 8 Bezug genommen, wo gleiche oder gleichwirkende Teile mit derselben, jedoch mit dem Buchstaben a versehenen Bezugszahl wie bei dem vorangehenden Ausführungsbeispiel bezeichnet sind.

Mit 18 ist in Fig. 8 ein zylindrischer Arretierungseinsatz bezeichnet, der in ein Verbindungsteil 1a eines Implantathohlkörpers mit Öffnungsschlitzen 4a und Abstandhalterflügeln 2a und 3a einführbar und in dem Verbindungsteil in der Art eines Zylinderschlosses drehbar ist. Von dem zylindrischen Einsatz 18 stehen diametral Hakenteile 19 vor.

Mit 20 ist eine stirnseitig an dem zylindrischen Einsatz 18 vorgesehene Sechskantausnehmung bezeichnet. Im Unterschied zu dem Implantathohlkörper gemäß Fig. 1 bis 6 weist der in Fig. 7 gezeigte Implantatkörper ein Gewinde 21 auf, das zum Teil in einer in einen der Schlitze 4a vorstehenden Nase 22 ausgebildet ist.

Der zylindrische Einsatz 18 ist in der in Fig. 8 gezeigten Stellung in den Implantathohlkörper einschiebbar, wobei sich die Hakenteile 19 innerhalb der Abstandhalterflügel 2a und 3a bewegen.

Durch eine Stirnwand 7a ist ein Anschlag für den zylindrischen Einsatz 18 gebildet.

Durch Drehung des zylindrischen Einsatzes 18 mit Hilfe eines in die Sechskantausnehmung 20 eingreifenden Sechskantschlüssels können die Hakenteile 19 in die in Fig. 7 gezeigte Position bewegt werden, in der sie durch die Schlitze 4a hindurch und von dem Implantathohlkörper vorstehen. Bei Anordnung zwischen zwei Wirbeln in der in Fig. 5 und 6 gezeigten Weise ragen sie unter Verhakung in das Knochenweichgewebe hinein. Abschließend kann der in die Stellung von Fig. 7 gedrehte zylindrische Einsatz 18 innerhalb des Implantathohlkörpers mit Hilfe einer in das Gewinde 21 einschraubbaren, vorzugsweise kopflosen Schraube fixiert werden. Die Fixierung könnte einfacher auch durch eine Verklemmung des Einsatzes in dem Implantathohlkörper erfolgen, indem z.B. die Hakenteile 19 Führungsanschläge zur Führung in den Schlitzen 4a aufweisen.

Die Fixierung kann so erfolgen, daß ein gewisses Spiel des zylindrischen Einsatzes 18 innerhalb des Implantathohlkörpers verbleibt, so daß durch geringfügige durch dieses Spiel mögliche Bewegungen der Hakenteile 19 das Knochenwachstum angeregt wird.

Durch die in das Knochenweichgewebe eingreifenden Hakenteile 19 ist der Implantathohlkörper zwischen den Wirbeln in Richtung senkrecht zur Wirbelsäulenlängsachse festgelegt, wodurch für eine zusätzliche Stabilisierung der Wirbelsäule gesorgt ist.

In dem gezeigten Ausführungsbeispiel ist die Breite der Hakenteile 19 deutlich kleiner als die Breite der Schlitze 4a, so daß genügend Zwischenraum zum Einwachsen von Knochenverbindungsgewebe gegeben ist, das im vorliegenden Fall den zylindrischen Einsatz umwachsen kann. Es ist aber auch denkbar, den zylindrischen Einsatz selbst mit Öffnungen und Hohlräumen zu versehen, in welche Knochenverbindungsgewebe hineinwachsen kann.

Im Unterschied zu den gezeigten Hakenteilen 19 könnten Hakenteile mit seitlichen Schneiden vorgesehen sein, die ein Eindringen der Hakenteile in das Knochenweichgewebe bei der Drehung des zylindrischen Einsatzes 18 erleichtern.

Um zusätzlichen Raum für durchwachsendes Knochengewebe zu schaffen, kann der Einsatz auch an mehreren Stellen durchbrochen sein.

## Patentansprüche

1. Vorrichtung zur Verbindung von Wirbeln (8,9) der Wirbelsäule über ein zwischen den Wirbeln (8,9) anzuordnendes Implantat, das wenigstens einen Hohlraum und Öffnungen (4-6) zur Durchsetzung des Implantats mit zur Verbindung der Wirbel (8,9) gebildetem Knochengewebe aufweist, wobei das Implantat einen hohlen, im wesentlichen rotationssymmetrischen Verbindungsteil (1)für die Anlage gegen freigelegtes Knochenweichgewebe mit einer sich senkrecht zur Wirbelsäulenlängsachse erstreckenden Symmetrieachse aufweist, von dem sich diametral Abstandhalterflügel (2,3) für die Anlage gegen Knochenhartgewebe erstrecken,
wobei der Verbindungsteil (1) auf seinen dem Knochengewebe zugewandten Seiten jeweils mehrere, sich in ihrer Längsausdehnung senkrecht zu der Symmetrieachse erstreckende Öffnungsschlitze (4) aufweist,
**dadurch gekennzeichnet,**
**dass** sich der Hohlraum und
die Öffnungsschlitze (4) über den Verbindungsteil (1)hinaus auf die Abstandhalterflügel (2,3) erstrecken.

2. Vorrichtung nach Anspruch 1,
**dadurch gekennzeichnet,**
**dass** sich der Verbindungsteil (1) und die Abstandhalterflügel 1 (2,3) entsprechend geneigt zueinander angeordneten Wirbeln (8,9) verjüngen.

3. Vorrichtung nach einem der Ansprüche 1 oder 2,
**dadurch gekennzeichnet,**
**dass** das Implantat als Hohlkörper mit in der Art eines Käfigs durchbrochenen Wänden ausgebildet ist.

4. Vorrichtung nach einem der Ansprüche 1 bis 3,
**dadurch gekennzeichnet,**
**dass** die Abstandhalterflügel (2,3) an ihren Flügelenden mit Durchbrüchen (5) versehen sind.

5. Vorrichtung nach einem der Ansprüche 1 bis 4,
**dadurch gekennzeichnet,**
**dass** der Hohlkörper auf einer Stirnseite eine insbesondere mit Durchbrüchen (6) versehene Stirnwand (7) aufweist und auf einer der Stirnseite gegenüberliegenden Stirnseite offen ist.

6. Vorrichtung nach einem der Ansprüche 1 bis 5,
**dadurch gekennzeichnet,**
**dass** eine Arretierungseinrichtung (18-20) zur Festlegung des Implantats quer zur Wirbelsäulenlängsrichtung vorgesehen ist.

7. Vorrichtung nach Anspruch 6,
**dadurch gekennzeichnet,**
**dass** die Arretierungseinrichtung einen in das Implantat einführbaren Einsatz (18) mit einer Verhakungseinrichtung (19), insbesondere für den Eingriff in das Knochenweichgewebe, umfasst.

8. Vorrichtung nach Anspruch 7,
**dadurch gekennzeichnet,**
**dass** die Verhakungseinrichtung Hakenteile (19) umfaßt, die an einem in der Art eines Zylinderschlosses in dem Implantat drehbaren Einsatzkörper (18) angebracht sind.

9. Vorrichtung nach Anspruch 8,
**dadurch gekennzeichnet,**
**dass** die Hakenteile (19) durch die Öffnungsschlitze (4a) hindurch in das Knochenweichgewebe eindrehbar sind.

10. Vorrichtung nach einem der Ansprüche 7 bis 9,
**dadurch gekennzeichnet,**
**dass** der Arretierungseinsatz (18) in dem Implantat zwischen der Stirnwand (7a) und einer an der gegenüberliegenden Stirnseite anbringbaren Sperre festlegbar ist.

11. Vorrichtung nach Anspruch 10,
**dadurch gekennzeichnet,**
**dass** die Sperre eine in dem Implantat versenkbare, kopflose Schraube umfasst.

12. Vorrichtung nach einem der Ansprüche 7 bis 11,
**dadurch gekennzeichnet,**
**dass** der Arretierungseinsatz (18) in dem Implantat mit einem vorgegebenen Spiel festlegbar ist.

## Claims

1. Device for connecting vertebrae (8, 9) of the spinal column via an implant which is to be placed between the vertebrae (8, 9) and which has at least one hollow space and openings (4-6) allowing bone tissue to grow through the implant in order to connect the vertebrae (8, 9), said implant having a hollow and substantially rotationally symmetrical connecting part (1) which is designed to bear against exposed soft bone tissue and which has an axis of symmetry extending perpendicular to the longitudinal axis of the spinal column, and having spacer wings (2, 3) that extend diametrically from the connecting part (1) in order to bear against hard bone tissue, the connecting part (1) having, on each of its sides directed towards the bone tissue, several slit openings (4) whose lengthwise extent runs perpendicular to the axis of symmetry, **characterized in that** the hollow space and the slit openings (4) extend beyond the connecting part (1) onto the spacer wings (2, 3).

2. Device according to Claim 1, **characterized in that** the connecting part (1) and the spacer wings (2, 3) taper in a manner corresponding to vertebrae (8, 9) arranged at an inclination relative to each other.

3. Device according to one of Claims 1 and 2, **characterized in that** the implant is designed as a hollow body with walls perforated in the manner of a cage.

4. Device according to one of Claims 1 to 3, **characterized in that** the spacer wings (2, 3) are provided with holes (5) at their wing ends.

5. Device according to one of Claims 1 to 4, **characterized in that** one end face of the hollow body has an end wall (7) provided in particular with holes (6), while an opposite end face thereof is open.

6. Device according to one of Claims 1 to 5, **characterized in that** a locking means (18-20) is provided for securing the implant transversely with respect to the longitudinal direction of the spinal column.

7. Device according to Claim 6, **characterized in that** the locking means comprises an insert (18), which can be fitted into the implant and which has a hooking arrangement (19) designed particularly for engagement in the soft bone tissue.

8. Device according to Claim 7, **characterized in that** the hooking arrangement comprises hook parts (19) mounted on an insert body (18) that is rotatable in the manner of a cylinder lock within the implant.

9. Device according to Claim 8, **characterized in that** the hook parts (19) can be turned through the slit openings (4a) into the soft bone tissue.

10. Device according to one of Claims 7 to 9, **characterized in that** the locking insert (18) can be secured in the implant between the end wall (7a) and a blocking means mountable on the opposite end face.

11. Device according to Claim 10, **characterized in that** the blocking means comprises a headless screw that can be recessed within the implant.

12. Device according to one of Claims 7 to 11, **characterized in that** the locking insert (18) can be secured in the implant with a predetermined play.

## Revendications

1. Dispositif pour joindre des vertèbres (8, 9) de la colonne vertébrale via un implant à agencer entre les vertèbres (8, 9), qui présente au moins une cavité et des ouvertures (4-6) pour la traversée de l'implant avec du tissu osseux qui se forme pour la liaison des vertèbres (8, 9), l'implant comprenant une partie de liaison creuse (1), sensiblement à symétrie de révolution, pour venir en appui contre du tissu osseux mou dégagé et présentant un axe de symétrie qui s'étend perpendiculairement à l'axe longitudinal de la colonne vertébrale, depuis lequel s'étendent diamétralement des ailettes d'écartement (2, 3) pour la venue en appui contre le tissu osseux dur,
et dans lequel la partie de liaison (1) comporte, sur ses côtés tournés vers le tissu osseux, plusieurs fentes d'ouverture respectives (4) qui s'étendent quant à leur extension longitudinale perpendiculairement à l'axe de symétrie,
**caractérisé en ce que** la cavité et les fentes d'ouverture (4) s'étendent au-delà de la partie de liaison (1) vers les ailettes d'écartement (2, 3).

2. Dispositif selon la revendication 1,
**caractérisé en ce que** la partie de liaison (1) et les ailettes d'écartement (2, 3) vont en se rétrécissant de manière correspondante à des vertèbres (8, 9) agencées inclinées l'une vers l'autre.

3. Dispositif selon l'une des revendications 1 ou 2,
**caractérisé en ce que** l'implant est réalisé sous forme de corps creux avec des parois présentant des percées à la manière d'une cage.

4. Dispositif selon l'une des revendications 1 à 3,
**caractérisé en ce que** les ailettes d'écartement (2, 3) sont dotées de percées (5) à leurs extrémités.

5. Dispositif selon l'une des revendications 1 à 4,
**caractérisé en ce que** le corps creux présente sur une face frontale une paroi frontale (7) en particulier dotée de percées (6), et **en ce qu'**il est ouvert sur un côté frontal opposé à ladite face frontale.

6. Dispositif selon l'une des revendications 1 à 5,
**caractérisé en ce qu'**il est prévu des moyens d'arrêt (18-20) pour immobiliser l'implant transversalement à la direction longitudinale de la colonne vertébrale.

7. Dispositif selon la revendication 6,
**caractérisé en ce que** les moyens d'arrêt comprennent un insert (18) susceptible d'être introduit dans l'implant avec un dispositif d'accrochage (19), en particulier pour l'engagement dans le tissu osseux mou.

8. Dispositif selon la revendication 7,
**caractérisé en ce que** le dispositif d'accrochage comprend des pièces en crochet (19) qui sont montées sur un corps d'un insert (18) capable de tourner dans l'implant à la manière d'une serrure à cylindre.

9. Dispositif selon la revendication 8,
**caractérisé en ce que** les pièces en crochet (19) peuvent être introduites par rotation dans le tissu osseux mou à travers les fentes d'ouverture (4a).

10. Dispositif selon l'une des revendications 7 à 9,
**caractérisé en ce que** l'insert d'arrêt (18) peut être immobilisé dans l'implant entre la paroi frontale (7a) et un verrou susceptible d'être monté sur la face frontale opposée.

11. Dispositif selon la revendication 10,
**caractérisé en ce que** le verrou comprend une vis sans tête à enfoncer dans l'implant.

12. Dispositif selon l'une des revendications 7 à 11,
**caractérisé en ce que** l'insert d'arrêt (18) peut être immobilisé dans l'implant avec un jeu prédéterminé.
